# EUROPEAN PATENT SPECIFICATION

(11) **EP 3 242 699 B1**
(45) Date of publication and mention of the grant of the patent: **10.03.2021**
(21) Application number: 16700259.1
(22) Date of filing: 08.01.2016
(51) Int. Cl.: A61M 5/315, H04B 5/00, A61M 5/24

(54) **A WIRELESS DATA COMMUNICATION MODULE FOR DRUG INJECTION DEVICES**
DRAHTLOSES KOMMUNIKATIONSMODUL FÜR ARZNEIMITTELINJEKTIONSVORRICHTUNGEN
MODULE DE COMMUNICATION DE DONNÉES SANS FIL POUR DISPOSITIFS D'INJECTION DE MÉDICAMENTS

(30) Priority: 08.01.2015 EP 15150512
(43) Date of publication of application: 15.11.2017
(73) Proprietor: Novo Nordisk A/S, 2880 Bagsværd (DK)
(72) Inventor: ROLSTED, Niels Pryds, 2880 Bagsværd (DK); HANSEN, Kim Ejholm, 2880 Bagsværd (DK); BORGHI, Tommaso, 20122 Milano (IT); PEDERSEN, Bennie, 2880 Bagsværd (DK); BORREGAARD, Sune Pelle, 2880 Bagsværd (DK); VALSECCHI, Luca, 20126 Milano (IT); SORO, Paolo, 20125 Milano (IT); BERNARDINI, Lorenzo, 20135 Milano (IT)
(86) International application number: PCT/EP2016/050320
(87) International publication number: WO 2016/110592

(56) References cited:
- WO-A1-2014/128156
- WO-A1-2014/161952
- KR-A- 20140 134 238
- US-A1- 2014 159 848
- US-A1- 2014 370 804
- "Vikuiti Product Sheet", , 31 December 2010 (2010-12-31), XP055204395, Retrieved from the Internet: URL:http://multimedia.3m.com/mws/media/374 730O/vikuiti-tm-esr-sales-literature.pdf?f n=ESR ss2.pdf [retrieved on 2015-07-23]

## Description

The present invention relates to a wireless data communication module for a drug injection device. The wireless data communication module comprises a folded flexible carrier member comprising a plurality of stacked component support regions and a display, such as a LCD or OLED display, electrically connected to the first component support region of the foldable carrier member via a first set of electrical connection terminals. The display comprises an outwardly facing readable display and an opposing, downwardly facing, optical reflector. The wireless data communication module additionally comprises a NFC antenna attached to a second component support region of the folded flexible carrier member situated below the first component mounting region. The optical reflector of the display comprises an optically reflective and non-magnetically permeable material.

### BACKGROUND OF THE INVENTION

The present invention relates to a wireless data communication module for drug injection or administration devices. Drug injection devices have greatly improved the lives of patients who must self-administer drugs or biological agents. Drug injection devices are also of great utility for care givers in administering injectable medicines to patients which for various reasons are incapable of performing self-injections. Performing a necessary insulin injection to a diabetes patient at the correct time and in the right dose size is essential for managing diabetes, i.e. it is important that the patient is in compliance with the specified insulin regimen. Drug injection devices may take many forms, including simple disposable devices that are little more than an ampoule with an injection means or they may be durable devices adapted to be used with pre-filled cartridges. Drug injection devices often comprise a pen shaped housing structure with a relatively small outer diameter such that the patient can conveniently grip and manipulate the drug injection device in connection with self-administration of the drug or biological agent in question.

To allow medical personnel to determine the effectiveness of a prescribed dosage regime or pattern, diabetes patients are often encouraged to keep a log of the size and time of each insulin injection. However, such data logs are normally kept in handwritten notebooks such that the logged information may not be easily uploaded to a computer for data processing. Furthermore, as only events, which are noted by the patient, are logged, the note book system requires that the patient remembers to log each injection, if the logged information is to have any value in the treatment of the patient's disease. A missing or erroneous record in the log results in a misleading picture of the injection history and thus a misleading basis for the medical personnel's decision making with respect to future medication. Consequently it is generally desirable to automate logging of dosage administration or injection information of drug injection devices by including suitable data recoding electronics of the drug injection devices. Furthermore, to conveniently transfer the acquired dosage administration or injection data to an associated computerized treatment system or component at the hospital or at a doctor's clinic, it is advantageous to include a wireless data transmitter or transceiver in the drug injection device. The acquired dosage administration or injection data may also be transferred to an intermediary portable data communication device such as a smartphone or tablet. The latter devices may be connected to the computerized treatment system via a wired or wireless data communication link for example including an internet connection. The wireless data transmitter or transceiver of the drug injection device may operate according to standardized data communication protocol such as Near Field Communication (NFC) as defined by the NFC Forum to establish a wireless data communication link to the computerized treatment system. However, drug injection devices such as a pen shaped device are subjected to severe limitations with respect to the overall size/diameter of a housing of the drug injection device to provide an appealing and sellable design. Hence, the electronic and mechanical components housed in the drug injection device are subjected to the same size constraints. These size constraints combined with the desirable integration of electronic dose logging electronics, a battery, a display and wireless data communication capabilities lead to various interoperability problems, in particular a size and placement of a wireless antenna for wireless data communication.

WO 2014/128156 A1 discloses a sensor assembly comprising first and second portions and an NFC antenna for integration in a drug delivery device. The first portion comprises a first rotary sensor part with a surface with a plurality of individual electrically conducting sensor areas arranged in a pattern. The second portion comprises a second rotary sensor part arranged rotationally relative to the first portion thereby defining an axis of rotation. The second rotary sensor part comprising a plurality of contact structures adapted to be in contact with conducting sensor areas on the first sensor rotary part.

The present invention provides a NFC based wireless data communication module in a compact module size without compromising data transmission distance and display readability. In the following disclosure aspects and embodiments of the present invention will be described which address one or more of the above objects or which address objects apparent from the disclosure as well as from the description of exemplary embodiments.

### SUMMARY OF INVENTION

A first aspect of the invention, which is defined in the independent claim 1, relates to a wireless data communication module for a drug injection device. The wireless data communication module comprises a folded flexible carrier member comprising a plurality of stacked component support regions, and foldable sections connecting two or more of the plurality of stacked component support regions to each other. The wireless data communication module further comprises a display, such as a LCD or OLED display, electrically connected to a first component support region of the folded flexible carrier member via a first set of electrical connection terminals. The display comprises an outwardly facing readable display and an opposing, downwardly facing, optical reflector. The wireless data communication module additionally comprises a NFC antenna attached to a second component support region of the folded flexible carrier member situated below the first component support region. An electronics circuit assembly of the wireless data communication module comprises at least a processor and a non-volatile memory, where the electronics circuit assembly is attached to a third component support region of the folded flexible carrier member situated below the second support region. The optical reflector of the display comprises an optically reflective and non-magnetically permeable material. A magnetically permeable sheet, such as a ferrite sheet, is situated in-between the NFC antenna on the second component support region and the electronics circuit assembly on the third component support region shielding the NFC antenna from EMI noise generated by the electronics circuit assembly.
The non-magnetically permeable material of the optical reflector preferably has a relative permeability µᵣ of less than 1.5, or less than 1.1, such as 1.0 or less.

The non-magnetically permeable material of the optical reflector allows the NFC antenna to be situated below the display LCD or OLED display without causing significant attenuation of magnetically carried or transmitted data signals between the NFC antenna and a receiving device such as a NFC enabled portable communication terminal e.g. a smartphone or tablet. This feature allows an area of the display LCD or OLED display to be larger than or equal to an area of the NFC antenna without causing unacceptable attenuation of the magnetically carried data signals due to an electromagnetic shadow effect of the optical reflector when the NFC antenna is situated below in the folded or stacked configuration of the first, second and third component support regions of the wireless data communication module. Since typical drug injection devices are subjected to severe housing size constraints as discussed above, it is advantageous to provide the largest possible area of the LCD or OLED display to improve its readability. This is achieved by the present wireless data communication module because of the non-magnetically permeable material of the optical reflector. The non-magnetically permeable material of the optical reflector may additionally be non-metallic and for example comprise a reflective display film as discussed in additional detail below with reference to the appended drawings.

The non-magnetically permeable optical reflector of the LCD or OLED display may possess a predetermined reflector area delimited by an outer circumferential reflector edge. The NFC antenna may have a predetermined antenna area delimited by an outer circumferential antenna edge. The predetermined antenna area may be smaller than the predetermined reflector area to support a display with large dimensions even when the NFC antenna is aligned below the LCD or OLED display in a stacked configuration of module components as discussed above. The NFC antenna is preferably substantially flat and the outer circumferential antenna edge may have various shapes such as circular, rectangular, elliptical etc. The predetermined antenna area is preferably smaller than 314 mm², which corresponding to an antenna diameter of 20 mm, or more preferably smaller than 180 mm² corresponding to an antenna diameter of about 15 mm to allow integration into a modest sized housing structure or member of the wireless data communication module. The housing member of the wireless data communication module may surround and protect the folded flexible carrier member and the components mounted thereon from the external environment. The LCD or OLED display may be attached at an aperture of a proximal end of the housing member with the outwardly facing readable display projecting away from the housing structure. One embodiment of the housing member comprises a cylindrical housing structure comprising a central longitudinal axis, a proximal annular end and a distal, opposing, annular end. The NFC antenna may be arranged below the non-magnetically permeable optical reflector along the central longitudinal axis such that the outer circumferential reflector edge completely overlaps the outer circumferential antenna edge.

The cylindrical housing structure makes the present wireless data communication module well-suited for integration in a pen shaped drug injection device. The cylindrical housing structure may have a diameter between 15 mm and 30 mm, even more preferably between 17 mm and 25 mm, to accommodate integration in ordinary pen shaped drug injection devices. In the present context the diameter is defined as the largest diameter of the cylindrical housing structure measured between its outer wall surfaces.

The folded flexible carrier member may possess numerous different structures. The folded flexible carrier member may comprise a Flexible Printed Circuit for example a flexible printed circuit board where the plurality of component support regions is integrally formed. The flexible printed circuit may be formed as a single coherent structure providing mechanical support of the components, e.g. the display, the NFC antenna, the electronics assembly etc., at the respective component support regions, and establishing electrical interconnections between the relevant components of the wireless module. Each of the component support regions of the flexible printed circuit board may be more rigid than other foldable sections connecting two or more rigid component support regions to each other. An alternative embodiment of the folded flexible carrier member comprises a plurality of rigid printed circuit board sections, or ceramic substrate sections, comprising the plurality of component support regions. The plurality of printed circuit board or ceramic substrate sections may be mechanically and electrically interconnected using a plurality of flexible and foldable conductor members. Each of the flexible and foldable conductor members may comprise a flexible printed circuit board with appropriate electrical traces or wires.

The NFC antenna may comprise a plurality of coil windings formed as wire traces of the second component support region of the folded flexible carrier member to provide a substantially flat structure of the NFC antenna. The NFC antenna may for example comprise between 4 and 10 individual coil windings. One embodiment of the NFC antenna comprises a first set of coil windings formed on a first surface of the second component support region and second set of coil windings formed on a second surface of the second component support region wherein the second surface is arranged oppositely to the first surface on the second component support region. Each coil winding of the first set of coil windings is preferably aligned with a coil winding of the second set of coil windings. Additional properties and embodiments of the NFC antenna are discussed in further detail below with reference to the appended drawings.

In accordance with the invention, the wireless data communication module comprises a magnetically permeable sheet, such as a ferrite sheet, situated in-between the NFC antenna and the electronics circuit assembly. This is achieved in an embodiment where the magnetically permeable sheet is attached to a fourth component support region of the folded flexible carrier member where the fourth component support region is situated in-between the second and third component support regions shielding the NFC antenna from EMI noise generated by the electronics circuit assembly. The magnetically permeable sheet may additionally be electrically conductive, e.g. comprising ferrite, to attenuate electrical field components of the EMI noise emitted by the electronics circuit assembly. A spacer may be attached to a first surface of the magnetically permeable sheet and the spacer may comprise an adhesive agent or foil for attachment to the fourth component support region of the folded flexible carrier member. The magnetically permeable sheet and spacer may form part of a separate EMI shielding module. The separate EMI shielding module may be reflow soldering compatible to support surface mount technology (SMT) based manufacturing of the flexible carrier member as discussed in further detail below with reference to the appended drawings.

In some embodiments, the magnetically permeable sheet is attached to the second component support region of the folded flexible carrier member, wherein the second component support region is situated in-between the first and third component support regions shielding the NFC antenna from EMI noise generated by the electronics circuit assembly.

In further embodiments, the second component support region and the fourth component support region may be located on opposing surfaces of a common carrier segment of the folded flexible carrier member, preferably such that the magnetically permeable sheet and the substantially flat NFC antenna are aligned below each other. The common carrier segment may comprise a double sided Flexible Printed Circuit or a double-sided rigid PCB.

A second aspect of the invention relates to a drug injection device, in particular a pen shaped drug injection device. The drug injection device comprises a pen shaped housing structure having a cylindrical hollow body member extending between a distal pen section and a proximal pen section, where the proximal pen section comprises a housing cut-out for receipt of a wireless data communication module according to any of the above-described embodiments thereof mounted in the housing cut-out as discussed in additional detail below with reference to the appended drawings.

In some embodiments, the pen shaped housing structure may be fully or partly made of a metal, such as a steel alloy or aluminium. In other embodiments, the pen housing structure may be made from plastic, with or without a metallized outer surface. In some embodiments, the external diameter of the pen housing structure, at the proximal end carrying the wireless data communication module, may be in the order of 14-20 mm, such as in the order of 15-16 mm.

In some embodiments, the wireless data communication module is arranged in a module housing structure which extends beyond an end surface of the housing structure. The module housing structure may be configured axially movable as an injection button which is pushed towards the pen housing structure for injection of a set dose.

In some further embodiments the NFC antenna of the wireless data communication module is located spaced away with 8-13 mm axial distance, such as about 10mm axial distance from the proximal end of the pen housing structure, when the injection button is arranged in a pre-defined axial position relative to the pen housing structure. The device may be configured to only accept data transfer with the receiving device when the injection button assumes the predefined axial position. The pre-defined axial position may correspond to the position the injection button assumes when the button has been pushed in relative to the pen housing structure.

### BRIEF DESCRIPTION OF THE DRAWINGS

Preferred embodiments of the invention will be described in additional detail in connection with the appended drawings, in which:
FIG. 1 is a simplified axial cross-sectional view of a pen shaped wireless data communication module in accordance with a first embodiment of the invention,
FIG. 2 is a simplified schematic view of a flexible carrier member in an unfolded state for use in the wireless data communication module,
FIG. 3 is a simplified schematic view of a LCD display of the wireless data communication module,
FIG. 4 is a simplified schematic view of an EMI shielding module of the wireless data communication module, and
FIG. 5 is a simplified schematic perspective view of an exemplary drug delivery or drug injection device incorporating the wireless data communication module.

### DETAILED DESCRIPTION OF PREFERRED EMBODIMENTS

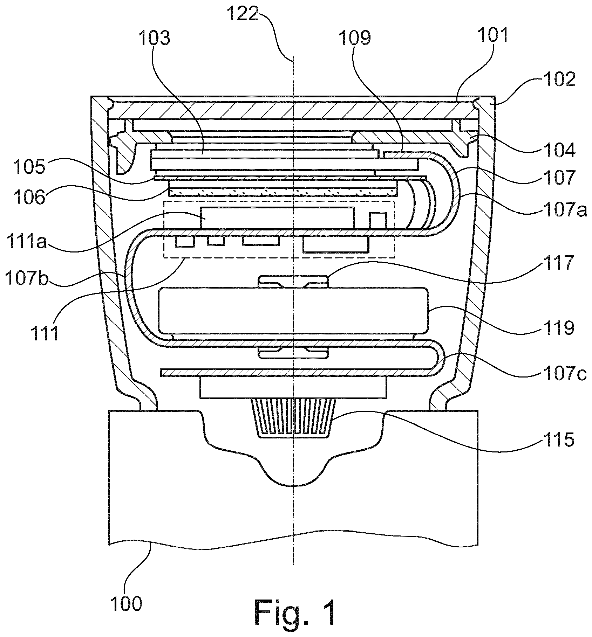

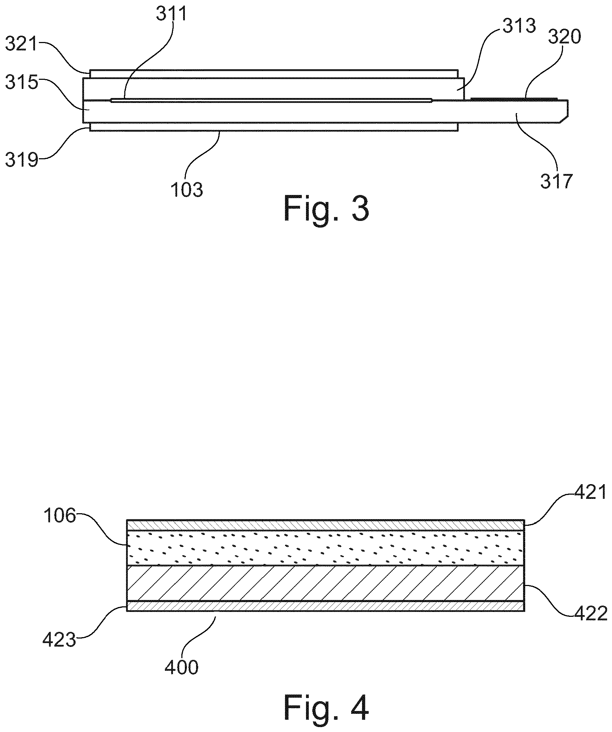

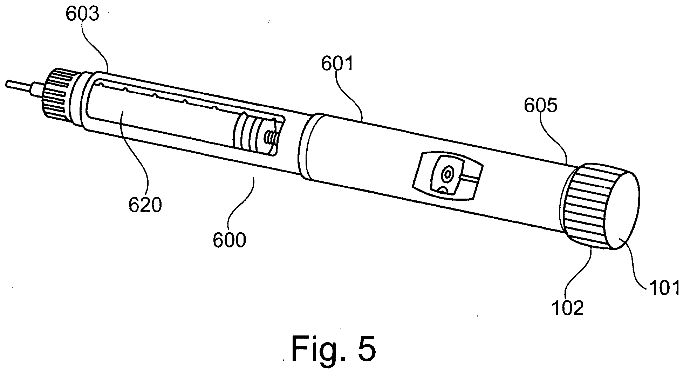

FIG. 1 shows a simplified axial cross-sectional view of a pen shaped wireless data communication module 100 suitable for use as a component or sub-assembly of a pen shaped drug injection device. The pen shaped wireless data communication module 100 comprises a substantially cylindrical housing structure 102 enclosing various electronic and other components of the wireless data communication module 100. The substantially cylindrical housing structure 102 comprises a proximal annular end wherein a display 103, such as a Liquid Crystal Display (LCD) or organic light-emitting diode (OLED) display, is mounted below a transparent display window 101. The transparent display window 101 is attached to an inner peripheral wall of the cylindrical housing structure 102 at the proximal annular end thereof. The transparent display window 101 may therefore seal and protect an interior volume of the cylindrical housing structure 102 against the external environment while allowing information on the LCD display to be read. As shown, a plurality of exposed sensor interface contacts or terminals 115 of a folded flexible carrier member 107 project from a distal, opposing, annular end of the cylindrical housing structure 102. As illustrated, the cylindrical housing structure 102 has small gradual tapering from the proximal annular end towards the distal annular end. The LCD display 103 comprises an outwardly facing readable display section (refer to items 311, 313 of FIG. 3) such that the user or patient is able to read information on the LCD display through the transparent window 101 when the wireless data communication module 100 is integrated in a drug delivery device as discussed in additional detail below with reference to FIG. 5.

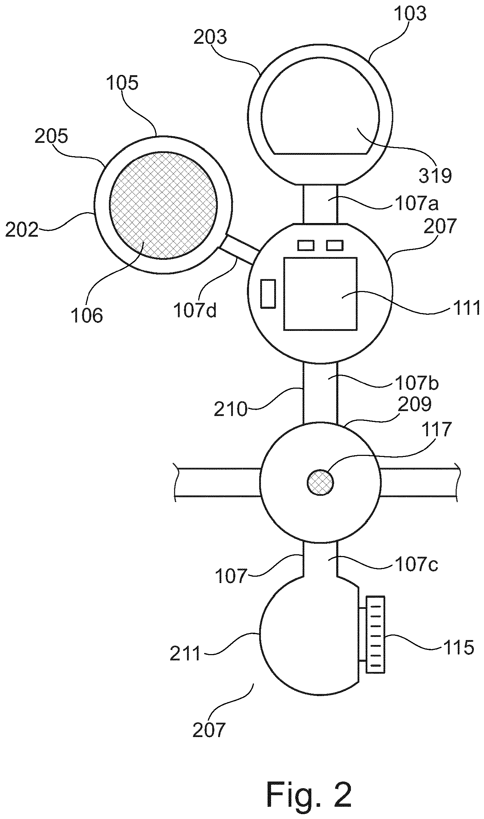

The folded flexible carrier member 107 comprises a plurality of stacked component support regions where electronic and mechanical components of the wireless data communication module 100 are mounted or attached. These component support regions are preferably relative plane as illustrated to adequately contact and support plane components mounted thereon. The stiffness of the plane component support regions of the folded flexible carrier member 107 may be higher than the stiffness of folded sections 107a, 107b, 107c mechanically and electrically interconnecting the plane component support regions. The flexibility of the folded sections 107a, 107b, 107c allows the flexible carrier member 107 to be folded into the folded structure. The flexible carrier member 107 may comprise a flexible printed circuit board or a flex-rigid printed circuit board. The skilled person will appreciate that the flexible carrier member 107 may be formed in alternative ways in other embodiments of the invention for example by a plurality of separate printed circuit board pieces/sections or ceramic carrier pieces/sections interconnected by various types of flexible and foldable conductor members such as electrical wires or leads. The separate printed circuit board or ceramic carrier pieces may comprise the plurality of component support regions. However, in the present embodiment of the invention, the flexible carrier member is formed by a Flexible Printed Circuit 107. The Flexible Printed Circuit 107 is in an unfolded state schematically illustrated on FIG. 2 as discussed below in further detail.

An electrical interface portion of the LCD display 103 is connected to a first component support region 109 of the Flexible Printed Circuit 107 just below display window support member 104. A substantially flat NFC antenna 105 is attached to a second component support region of the folded Flexible Printed Circuit 107 such that the flat NFC antenna 105 is situated below the LCD display 103 mounted on the first component mounting region in the folded state of the Flexible Printed Circuit 107. The wireless data communication module 100 further comprises an electronics circuit assembly 111 attached to a third component support region of the Flexible Printed Circuit 107 situated below the second support region due the 180 degrees bended folded section 107a of the Flexible Printed Circuit 107. The electronics circuit assembly 111 may comprise a digital processor such as a software programmable microprocessor, a clock circuit or clock generator and a non-volatile memory such as EPROM or EEPROM. The non-volatile memory may comprise an automatically collected data log or data record of the patient's drug administration using the drug injection device where the wireless module 100 is integrated. The electronics circuit assembly 111 preferably comprises a display driver for the LCD display 103 and a wireless antenna transmitter or transceiver electrically connected to the NFC antenna 105 such that the logged data can be wirelessly transmitted via the NFC protocol to an external NFC compatible device such as a smartphone or tablet forming part of a medical system. The electronics circuit assembly 111 and NFC antenna 105 may in addition be configured to receive various types of wireless data from the external NFC compatible device. The LCD display 103 comprises an optical reflector 319 which is opposing the outwardly facing transparent display portion 311, 313 discussed above. Hence, when the LCD display 103 is mounted in the wireless data communication module 100 as illustrated on FIG. 1, the optical reflector 319 is placed just above the NFC antenna 105. Due to the severely limited space in the interior of the housing structure 102 only a small axial separation may be provided between optical reflector 319 and NFC antenna 105. Furthermore, near-field NFC communication relies on the use of electromagnetic induction between two loop antennas to transmit data. Hereby the NFC antenna 105 is a first loop antenna configured for being inductively coupled with a second loop antenna when the two loop antennas are located within each other's near field. Hence, the presence of magnetically permeable or conducting material between the NFC antenna 105 and the NFC antenna of the receiving device, such as a smartphone or tablet etc., will attenuate the transmitted magnetic signal. This magnetic signal attenuation is particularly severe when an area of the optical reflector 319 is similar to, or larger than, an area of the NFC antenna 105 because the electromagnetic shielding effect becomes more effective. The amount of signal attenuation may be sufficient to make data communication between the NFC antenna 105 and the receiving antenna impossible or highly unreliable.

In that connection it is also noticeable that the electronics circuit assembly 111 may emit various types of electromagnetic noise, for example due to the clock signals of the digital processor, which interfere with the wireless communication channel between the NFC antenna 105 and the receiving antenna. This problem has been addressed and solved by the present inventors by the provision of a customized LCD display 103 where the material of the optical reflector is formed by, or comprises, a non-magnetically permeable and optical reflective material such as an optically reflective plastic film. The customized LCD display 103 comprises an optical reflector 319 without the ordinary metallic and magnetically permeable optical reflector materials. One embodiment of the optical reflector 319 comprises a Vikuiti™ Reflective or Transreflective Display Film, such as a black RDF-B type manufactured by 3M Electronic Display Lighting Optical Systems Division. These films exhibit good optical properties in addition to the non-metallic and non-magnetically permeable properties.

The Flexible Printed Circuit 107 of the wireless data communication module 100 additionally comprises a magnetically permeable sheet, for example a ferrite disc 106 attached to a fourth component support region of the folded Flexible Printed Circuit 107. The fourth component support region is situated in-between the second and third component support regions such that the substantially flat NFC antenna is electromagnetically shielded from the previously discussed EMI noise generated by the electronics circuit assembly 111. The ferrite disc 106 is part of an EMI shielding module 400 illustrated on FIG. 4 in further detail. The electrically conductive and magnetically permeable properties of the ferrite disc 106 are effective in reflecting and/or absorbing the EMI noise emitted by the electronics circuit assembly 111. The EMI shielding module 400 (refer too FIG. 4) may comprise a non-metallic spacer 422 and an adhesive foil 421 attached to a first surface of the spacer. The adhesive foil 421 is used to attach the EMI shielding module 400 to the fourth component support region. The second and fourth component support regions of the folded Flexible Printed Circuit 107 are located on opposing surfaces of a common Flexible Printed Circuit portion or segment (item 202 of FIG. 2) of the folded flexible carrier member 107. As illustrated, the ferrite disc 106 and the NFC antenna 105 are substantially aligned below each other, albeit separated by the non-metallic spacer 422, along the central axis 122 of the module 100.

The Flexible Printed Circuit 107 of the wireless data communication module 100 additionally comprises a battery 119 attached to or abutting a fifth component support region of the folded Flexible Printed Circuit 107 via a pair of battery connectors 117. The fifth component support region is placed below the fourth component support region via another folded section 107b of flexible carrier member 107. The battery 119 may comprise one or more rechargeable or non-rechargeable battery cell(s) depending on the particular application of the wireless data communication module 100. The battery 119 may supply power to active components and circuits, such as the microprocessor 111a etc., of the electronics circuit assembly 111 via conductive wire traces of the folded Flexible Printed Circuit 107 running through the folded section 107b. A sixth component support region of the folded Flexible Printed Circuit 107 is located at termination portion thereof and comprises the previously discussed exposed sensor interface contacts or terminals 115. These exposed sensor interface contacts or terminals 115 may be formed integrally on the Flexible Printed Circuit 107 for example as exposed wire ends. The exposed sensor interface contacts 115 may be used to establish electrical connection to various types of sensors of the drug injection device for example a position encoder arrangement providing dose size information, a mode sensor for providing information whether the drug injection device is in dose setting mode or dose expelling mode, and an end of dose sensor providing a signal indicating finalisation of a dosage expelling procedure. Further sensors or electronic circuitry may also be envisaged, such as a cartridge sensor for recognizing a cartridge identifier, cartridge presence sensor etc.

Since the dimensions, including a radial dimension thereof, of the wireless data communication module 100 are severely limited, it is generally advantageous and desirable to make dimensions of the LCD display 103 as large as practically possible to enhance the readability of text and graphics to be displayed. This implies that the optical reflector likewise should be as large as practically possible. However, such large dimensions of the optical reflector have the drawback that the electromagnetic shielding effect of the magnetically carried data signals emitted, or received, by the NFC antenna 105 becomes more effective and attenuates the magnetically carried data signals. If the area of a magnetically permeable optical reflector is larger than the area of the NFC antenna, the attenuation of the magnetically carried data signals is high, in particular along the central longitudinal axis 122 of the module 100. However, in the present embodiment, the large dimensions of the LCD display 103 and its optical reflector 319 do not cause attenuation of the magnetically carried data signals thanks to the non-magnetically permeable material of the optical reflector 319. Therefore, it is unproblematic that the area of the optical reflector 319 of the LCD display 103 is larger than the area of the NFC antenna 105 in the present embodiment of the invention. In alternative embodiments of the module, the area of the optical reflector 319 may of course be equal to or smaller than the area of the NFC antenna 105 and still benefit from the lower attenuation of the magnetically carried data signals afforded by the non-magnetically permeable material of the optical reflector 319. In this context, the area of the NFC antenna 105 is delimited by an outer circumferential antenna edge defined by the outermost edge of a plurality of coil windings of the NFC antenna 105, except for the antenna ends or terminations. The NFC antenna 105 comprises a plurality of coil windings (not shown) formed as conductive wire traces on the second component support region of the folded Flexible Printed Circuit 107. This makes it possible to integrate the NFC antenna directly in the folded Flexible Printed Circuit 107 for example comprising conventional copper traces leading to a highly cost-efficient, robust and compact design of the NFC antenna 105. The NFC antenna 105 may comprise a coil with different number of individual coil windings depending on application specific requirements and practical constraints. In one embodiment, the NFC antenna 105 comprises between 2 and 10 coil windings. The plurality of coil windings may be distributed between first and second oppositely arranged surfaces of the second component support region of the folded Flexible Printed Circuit 107 to utilize this support area in an efficient manner. Hence, first and second sets of coil windings may be formed on first and second surfaces, respectively, of the second component support region. Each of first and second sets of coil windings may comprise between 2 and 4 coil windings. In these double-sided implementations of the NFC antenna 105, the second component support region may comprise a double sided Flexible Printed Circuit section. Furthermore, each coil winding of the first set of coil windings may be aligned with a coil winding of the second set of coil windings to provide a compact antenna coil design providing a maximum area of the so-called Proximity Integrated Circuit Cards (PICC) antenna zone.

The area of the NFC antenna 105 is preferably smaller than 180 mm² in the present embodiment of the invention such that the antenna 105 can fit into the interior volume of the cylindrical housing structure 102 when oriented in a radial plane of the housing structure. The NFC antenna 105 preferably has a circular shape with a diameter of about 15 mm or smaller to optimize the antenna area to the inner cylindrical shape of the housing structure 102 of the module 100. The skilled person will appreciate that the antenna dimension is significantly smaller than the area of an ISO14443-1 Class 6 compliant circular antenna making it particularly challenging to obtain the ISO specified sensitivity and transmission distance of the NFC antenna 105. The width of each coil winding may lie between 0.70 mm and 0.50 mm and a thickness between 0.40 mm and 0.35 mm. The distance between individual coil windings of the antenna coil may be 0.1 mm or larger.

FIG. 2 shows a simplified schematic view of the Flexible Printed Circuit assembly 207 including the Flexible Printed Circuit 107 in an unfolded state and the above-discussed components mounted thereon. The Flexible Printed Circuit 107 comprises the previously discussed circular or semi-circular first (203), second (205), third (207), fourth (non-referenced), fifth (209) and sixth (211) component support regions integrally formed in the carrier material of the Flexible Printed Circuit. The first, second, third, fourth and fifth component support are electrically and mechanically interconnected via respective ones of the previously discussed folded, or foldable, sections 107a, 107b, 107c and 107d of the Flexible Printed Circuit 107. To obtain the stacked state of the Flexible Printed Circuit 107 depicted on FIG.1, the second component support region 205 is firstly folded against the third component support region 207 by folding section 107d such that the ferrite disc 106 faces the electronics circuit assembly 111 and the NFC antenna 105, arranged on the opposite side of the second component support region 205, faces upwardly. Thereafter, the first component support region 203 is folded against the second component support region 205 such that the underside of the LCD display 103, including the optical reflector 319, faces the NFC antenna 105 as discussed above.

The skilled person will appreciate that the Flexible Printed Circuit 107 and the electronic and other components mounted thereon preferably are SMT compatible. This allows the entire Flexible Printed Circuit assembly 207 to be assembled using conventional automated surface mount technology (SMT) processing. The EMI shielding module 400, including the ferrite disc 106, may be SMT compatible allowing a plurality of EMI shielding modules 400 to be placed on a conventional reel tape ready for SMT mounting onto the Flexible Printed Circuit 107.

FIG. 3 shows a simplified schematic view of the previously discussed LCD display 103. The LCD display 103 comprises stacked configuration of display components. The outwardly facing transparent display side comprises a liquid crystal member 311 arranged below a top glass 313. A polarizer 321 may be attached to the outwardly facing side of the top glass 313 as illustrated. The LCD display 103 further comprises a bottom glass member 315 arranged below (referring to the depicted orientation of the LCD display) the liquid crystal member 311. A contact portion or finger 317 of the bottom glass member 315 protrudes horizontally out from the stack of aligned display components comprising the polarizer 321, top glass 313, liquid crystal member 311 and optical reflector 319. The contact portion or finger 317 comprises a plurality of electrical display contacts 320 of the LCD display 103 for receipt of display data. These display data are transmitted from the previously discussed display driver of the electronics circuit assembly 111 via suitable data wires of the Flexible Printed Circuit 107. In some embodiments of the invention, suitably arranged interface terminals provided on a proximal end of the Flexible Printed Circuit 107 may be directly connected to the plurality of electrical display contacts 320 as schematically illustrated on FIG. 1. The lowermost portion of the LCD display 103 comprises the previously discussed reflective, non-metallic and non-magnetically permeable optical reflector 319.

FIG. 4 shows a simplified schematic view of the previously discussed EMI shielding module 400 comprising the ferrite disc 106. The EMI shielding module 400 is constructed such that it is compatible with reflow soldering processes used by conventional SMT mounting technologies utilized to manufacture the the Flexible Printed Circuit assembly 207 as discussed above. A protective layer 421 is mounted on a top surface of the ferrite disc 106 to protect the often rather brittle ferrite material from mechanical stress and impacts. In the present embodiment, the protective layer 421 is constructed to additionally withstand high temperature exposure in connection with reflow soldering which may involve temperatures at or above 260 degree C. The EMI shielding module 400 additionally comprises a spacer 422 which is attached to a lower surface of the ferrite disc 106. The spacer 422 preferably comprises a non-magnetically permeable material and creates a certain well-defined vertical (i.e. along the central axis of the module) distance to the NFC antenna 105 in the folded state of the Flexible Printed Circuit 107. The thickness of the spacer 422 may lie between 0.1 mm and 2 mm. The spacer 422 sets a certain distance between the NFC antenna 105 and the ferrite disc 106 such the magnetic properties of the latter does not interfere with the electromagnetic data signal emitted by the NFC antenna. An adhesive layer, agent or film 423 is attached to the surface of the spacer 423 facing away from the spacer 422. The adhesive layer, agent or film 423 may be used to attach the EMI shielding module 400 to the second component support region 205 of the Flexible Printed Circuit 107.

FIG. 5 is a simplified schematic view of an exemplary pen shaped drug injection device 600. The pen shaped drug injection device 600 comprises a housing structure with a cylindrical hollow body member 601 extending between a distal pen section 603 and a proximal pen section 605. A drug cartridge 620 is housed inside the cylindrical hollow body member 601. The proximal pen section 605 comprises a housing cut-out where the previously discussed wireless data communication module 100 is inserted or arranged. A distal outer wall section of the substantially cylindrical housing structure 102 of the module 100 fits into the housing cut-out of the cylindrical hollow body member 601 of the drug injection device 600. The LCD display is situated at the proximal annular end of the wireless data communication module 100 such that the transparent display window 101 is placed at the proximal end surface of the drug injection device 600.

In the shown embodiment, the module 100 serves as a dose setting an injection button. The dose setting and injection button is axially displaced away from an axial position in the housing as a dose is being dialled up. After a dose has been set, the button is axially pushed back to the original axial position to inject the set dose.

The wireless data communication module 100 may be electrically connected to various types of sensors, for example a dose sensor arrangement of the drug injection device 600 via the previously discussed exposed sensor interface contacts. In this manner, the non-volatile memory of the wireless data communication module 100 may be used to automatically collect the previously discussed data log or data record of the patient's drug administration. This data log may for example comprise the patient's injected drug doses and injection times. The content of the data log or record may be transmitted automatically to the external NFC enabled computing device via the NFC antenna either automatically or upon interrogation/request from the external NFC enabled computing device.

## Claims

1. A wireless data communication module (100) for a drug injection device, comprising:
- a folded flexible carrier member (107) comprising a plurality of stacked component support regions (203, 205, 207, 209, 211), and foldable sections (107a, 107b, 107c, 107d) connecting two or more of the plurality of stacked component support regions to each other,
- a display (103), such as a LCD or OLED display, electrically connected to conductors of a first component support region (203) of the folded flexible carrier member (107) via a first set of electrical connection terminals,
- where the display (103) comprises an outwardly facing readable display and an opposing, downwardly, facing optical reflector (319),
- a NFC antenna (105), and
- an electronics circuit assembly (111) comprising at least a processor and a non-volatile memory;
**characterized in that** the NFC antenna (105) is attached to a second component support region (205) of the folded flexible carrier member (107) situated below the first component support region (203),
wherein the electronics circuit assembly is attached to a third component support region (207) of the folded flexible carrier member (107) situated below the second support region (205),
wherein the optical reflector (319) of the display (103) comprises an optically reflective and non-magnetically permeable material, and
wherein a magnetically permeable sheet (106), such as a ferrite sheet, is situated in-between the NFC antenna (105) on the second component support region (205) and the electronics circuit assembly on the third component support region (207) shielding the NFC antenna (105) from EMI noise generated by the electronics circuit assembly.

2. A wireless data communication module according to claim 1, wherein the non-magnetically permeable material of the optical reflector (319) additionally is non-metallic.

3. A wireless data communication module according to claim 1 or 2, wherein the optical reflector (319) of the display (103) has a predetermined reflector area delimited by an outer circumferential reflector edge; and
the NFC antenna (105) has a predetermined antenna area delimited by an outer circumferential antenna edge,
wherein the predetermined antenna area is smaller than the predetermined reflector area.

4. A wireless data communication module according to claim 3, wherein the NFC antenna (105) is arranged below the optical reflector (319) along a longitudinal axis of the wireless data communication module (100) such that the outer circumferential reflector edge completely overlaps the outer circumferential antenna edge along the longitudinal axis.

5. A wireless data communication module for a drug injection device according to any of the preceding claims, wherein the folded flexible carrier member (107) comprises a flexible printed circuit board where the plurality of component support regions (203, 205, 207, 209, 211) are integrally formed.

6. A wireless data communication module for a drug injection device according to any of claims 1-4, wherein the folded flexible carrier member (107) comprises a plurality of printed circuit board sections comprising the plurality of component support regions (203, 205, 207, 209, 211), and
a plurality of flexible and foldable conductor members electrically interconnecting the plurality of printed circuit board sections.

7. A wireless data communication module according to any of claims 3 - 6, wherein the predetermined antenna area of the NFC antenna is smaller than 314 mm², more preferably smaller than 180 mm² (d=15 mm).

8. A wireless data communication module according to any of the preceding claims, wherein the NFC antenna (105) comprises a plurality of coil windings formed as wire traces of the second component support region (205) of the folded flexible carrier member (107) to provide a substantially flat structure of the NFC antenna.

9. A wireless data communication module according to claim 8, wherein the magnetically permeable sheet (106) and the substantially flat NFC antenna (105) are aligned relative to each other in an overlapping configuration.

10. A wireless data communication module according to any of the claims 8-9, wherein the plurality of coil windings comprises:
- a first set of coil windings formed on a first surface of the second component support region (205) and second set of coil windings formed on a second surface of the second component support region (205) wherein the second surface is arranged oppositely to the first surface on the second component support region (205).

11. A wireless data communication module according to any of the claims 1-10, wherein the magnetically permeable sheet (106) is attached to a fourth component support region of the folded flexible carrier member (107), wherein the fourth component support region is situated in-between the second component support region (205) and the third component support region (207).

12. A wireless data communication module according to any of the claims 1-9, wherein the magnetically permeable sheet (106) is attached to the second component support region (205) of the folded flexible carrier member (107), wherein the second component support region is situated in-between the first (203) and third (207) component support regions shielding the NFC antenna from EMI noise generated by the electronics circuit assembly.

13. A wireless data communication module according to claim 12, wherein a spacer (422) is attached to a first surface of the magnetically permeable sheet (106); and
the spacer comprises an adhesive agent or foil (423) for attachment to the second component support region (205) of the folded flexible carrier member (107).

14. A wireless data communication module according to any of the preceding claims, further comprising:
- a cylindrical housing structure (601) comprising a central longitudinal axis, a proximal annular end and a distal, opposing, annular end;
wherein the LCD or OLED display (319) is attached at the proximal annular end with the outwardly facing transparent display projecting away from the cylindrical housing structure (601).

15. A pen shaped drug injection device (600) comprising:
a pen shaped housing structure (601) having a cylindrical hollow body member extending between a distal pen section and a proximal pen section, where the proximal pen section comprises a housing cut-out for receipt of a wireless data communication, and
a wireless data communication module (100) according to claim 14 mounted in the housing cut-out.

## Patentansprüche

1. Drahtloses Datenkommunikationsmodul (100) für eine Arzneimittelinjektionsvorrichtung, umfassend:
- ein gefaltetes flexibles Trägerelement (107), das eine Vielzahl von gestapelten Komponentenauflagebereichen (203, 205, 207, 209, 211) und faltbare Abschnitte (107a, 107b, 107c, 107d), die zwei oder mehr aus der Vielzahl von gestapelten Komponentenauflagebereichen miteinander verbinden, umfasst
- eine Anzeige (103), wie z. B. eine LCD- oder OLED-Anzeige, die über einen ersten Satz von elektrischen Verbindungsterminals elektrisch mit Leitern eines ersten Komponentenauflagebereichs (203) des gefalteten flexiblen Trägerelements (107) verbunden ist,
- wobei die Anzeige (103) eine nach außen gerichtete ablesbare Anzeige und einen gegenüberliegenden nach unten gerichteten optischen Reflektor (319) umfasst,
- eine NFC-Antenne (105) und
- eine elektronische Schaltungsbaugruppe (111), die zumindest einen Prozessor und einen nicht-flüchtigen Speicher umfasst;
**dadurch gekennzeichnet, dass** die NFC-Antenne (105) an einem zweiten Komponentenauflagebereich (205) des gefalteten flexiblen Trägerelements (107) befestigt ist, der sich unter dem ersten Komponentenauflagebereich (203) befindet,
wobei die elektronische Schaltungsbaugruppe an einem dritten Komponentenauflagebereich (207) des gefalteten flexiblen Trägerelements (107) befestigt ist, der sich unter dem zweiten Komponentenauflagebereich (205) befindet,
wobei der optische Reflektor (319) der Anzeige (103) ein optisch reflektierendes und nicht magnetisch durchlässiges Material umfasst und
wobei sich eine magnetisch durchlässige Platte (106), wie z. B. eine Ferritplatte, zwischen der NFC-Antenne (105) auf dem zweiten Komponentenauflagebereich (205) und der elektronischen Schaltungsbaugruppe auf dem dritten Komponentenauflagebereich (207) befindet und die NFC-Antenne (105) vor EMI-Rauschen abschirmt, das durch die elektronische Schaltungsbaugruppe erzeugt wird.

2. Drahtloses Datenkommunikationsmodul nach Anspruch 1, wobei das nicht magnetisch durchlässige Material des optischen Reflektors (319) zusätzlich nicht-metallisch ist.

3. Drahtloses Datenkommunikationsmodul nach Anspruch 1 oder 2, wobei der optische Reflektor (319) der Anzeige (103) eine vorbestimmte Reflektorfläche aufweist, die durch eine äußere umfangsmäßige Reflektorkante begrenzt ist; und
die NFC-Antenne (105) eine vorbestimmte Antennenfläche aufweist, die durch eine äußere umfangsmäßige Antennenkante begrenzt ist,
wobei die vorbestimmte Antennenfläche kleiner ist als die vorbestimmte Reflektorfläche.

4. Drahtloses Datenkommunikationsmodul nach Anspruch 3, wobei die NFC-Antenne (105) unter dem optischen Reflektor (319) entlang einer Längsachse des drahtlosen Datenkommunikationsmoduls (100) angeordnet ist, sodass die äußere umfangsmäßige Reflektorkante die äußere umfangsmäßige Antennenkante entlang der Längsachse vollständig überschneidet.

5. Drahtloses Datenkommunikationsmodul für eine Arzneimittelinjektionsvorrichtung nach einem der vorhergehenden Ansprüche, wobei das gefaltete flexible Trägerelement (107) eine flexible gedruckte Leiterplatte umfasst, auf der die Vielzahl von Komponentenauflagebereichen (203, 205, 207, 209, 211) integral ausgebildet sind.

6. Drahtloses Datenkommunikationsmodul für eine Arzneimittelinjektionsvorrichtung nach einem der Ansprüche 1-4, wobei das gefaltete flexible Trägerelement (107) eine Vielzahl von Abschnitten einer gedruckten Leiterplatte, die die Vielzahl von Komponentenauflagebereichen (203, 205, 207, 209, 211) umfassen, und
eine Vielzahl von flexiblen und faltbaren Leiterelementen, die die Vielzahl von Abschnitten einer gedruckten Leiterplatte elektrisch miteinander verbinden, umfasst.

7. Drahtloses Datenkommunikationsmodul nach einem der Ansprüche 3-6, wobei die vorbestimmte Antennenfläche der NFC-Antenne kleiner als 314 mm² ist, besonders bevorzugt kleiner als 180 mm² (d = 15 mm).

8. Drahtloses Datenkommunikationsmodul nach einem der vorhergehenden Ansprüche, wobei die NFC-Antenne (105) eine Vielzahl von Spulenwindungen umfasst, die als Verdrahtungslinien des zweiten Komponentenauflagebereichs (205) des gefalteten flexiblen Trägerelements (107) ausgebildet sind, um eine im Wesentlichen flache Struktur der NFC-Antenne zu ermöglichen.

9. Drahtloses Datenkommunikationsmodul nach Anspruch 8, wobei die magnetisch durchlässige Platte (106) und die im Wesentlichen flache NFC-Antenne (105) in einer sich überschneidenden Konfiguration aneinander ausgerichtet sind.

10. Drahtloses Datenkommunikationsmodul nach einem der Ansprüche 8-9, wobei die Vielzahl von Spulenwindungen Folgendes umfasst:
- einen ersten Satz von Spulenwindungen, der auf einer ersten Oberfläche des zweiten Komponentenauflagebereichs (205) ausgebildet ist, und einen zweiten Satz von Spulenwindungen, der auf einer zweiten Oberfläche des zweiten Komponentenauflagebereichs (205) ausgebildet ist, wobei die zweite Oberfläche der ersten Oberfläche auf dem zweiten Komponentenauflagebereich (205) gegenüberliegend angeordnet ist.

11. Drahtloses Datenkommunikationsmodul nach einem der Ansprüche 1-10, wobei die magnetisch durchlässige Platte (106) an einem vierten Komponentenauflagebereich des gefalteten flexiblen Trägerelements (107) befestigt ist, wobei der vierte Komponentenauflagebereich sich zwischen dem zweiten Komponentenauflagebereich (205) und dem dritten Komponentenauflagebereich (207) befindet.

12. Drahtloses Datenkommunikationsmodul nach einem der Ansprüche 1-9, wobei die magnetisch durchlässige Platte (106) an dem zweiten Komponentenauflagebereich (205) des gefalteten flexiblen Trägerelements (107) befestigt ist, wobei sich der zweite Komponentenauflagebereich zwischen dem ersten (203) und dritten (207) Komponentenauflagebereich befindet und die NFC-Antenne vor EMI-Rauschen abschirmt, das durch die elektronische Schaltungsbaugruppe erzeugt wird.

13. Drahtloses Datenkommunikationsmodul nach Anspruch 12, wobei ein Abstandshalter (422) an einer ersten Oberfläche der magnetisch durchlässigen Platte (106) befestigt ist; und
der Abstandshalter ein Klebemittel oder eine Folie (423) zur Befestigung an dem zweiten Komponentenauflagebereich (205) des gefalteten flexiblen Trägerelements (107) umfasst.

14. Drahtloses Datenkommunikationsmodul nach einem der vorhergehenden Ansprüche, ferner umfassend:
- eine zylindrische Gehäusestruktur (601), die eine zentrale Längsachse, ein proximales ringförmiges Ende und ein distales, gegenüberliegendes ringförmiges Ende umfasst;
wobei die LCD- oder OLED-Anzeige (319) an dem proximalen ringförmigen Ende befestigt ist und die nach außen gerichtete transparente Anzeige von der zylindrischen Gehäusestruktur (601) abgewandt ist.

15. Stiftförmige Arzneimittelinjektionsvorrichtung (600), umfassend:
eine stiftförmige Gehäusestruktur (601), die ein zylindrisches Hohlkörperelement aufweist, das sich zwischen einem distalen Stiftabschnitt und einem proximalen Stiftabschnitt erstreckt, wobei der proximale Stiftabschnitt eine Aussparung zum Aufnehmen eines drahtlosen Datenkommunikationsmoduls und
ein in der Aussparung des Gehäuses montiertes drahtloses Datenkommunikationsmodul (100) nach Anspruch 14 umfasst.

## Revendications

1. Module de communication de données sans fil (100) pour un dispositif d'injection de médicament, comprenant :
- un élément porteur flexible plié (107) comprenant une pluralité de régions de support de composants empilés (203, 205, 207, 209, 211) et des sections pliables (107a, 107b, 107c, 107d) connectant deux ou plus de la pluralité de régions de support de composants empilés les un aux autres,
- un écran (103), tel qu'un écran LCD ou OLED, connecté électriquement aux conducteurs d'une première région de support de composants (203) de l'élément porteur flexible plié (107) via un premier ensemble de bornes de connexion électrique,
- où l'écran (103) comprend un écran lisible faisant face à l'extérieur et un réflecteur optique opposé, orienté vers le bas (319),
- une antenne NFC (105), et
- un ensemble de circuits électroniques (111) comprenant au moins un processeur et une mémoire non volatile ;
**caractérisé en ce que** l'antenne NFC (105) est fixée à une deuxième région de support de composant (205) de l'élément porteur flexible plié (107) situé sous la première région de support de composant (203),
dans lequel l'ensemble de circuits électroniques est fixé à une troisième région de support de composant (207) e l'élément porteur flexible plié (107) situé sous la deuxième région de support (205),
dans lequel le réflecteur optique (319) de l'écran (103) comprend un matériau optiquement réfléchissant et non perméable magnétiquement, et
dans lequel une feuille perméable magnétiquement (106), telle qu'une feuille de ferrite, est située entre l'antenne NFC (105) sur la deuxième région de support de composant (205) et l'ensemble de circuits électroniques sur la troisième région de support de composant (207), protégeant l'antenne NFC (105) du bruit EMI généré par l'ensemble de circuits électroniques.

2. Module de communication de données sans fil selon la revendication 1, dans lequel le matériau non perméable magnétiquement du réflecteur optique (319) est en outre non métallique.

3. Module de communication de données sans fil selon la revendication 1 ou 2, dans lequel le réflecteur optique (319) de l'écran (103) possède une zone de réflecteur prédéterminée délimitée par un bord de réflecteur circonférentiel externe ; et
l'antenne NFC (105) possède une zone d'antenne prédéterminée délimitée par un bord d'antenne circonférentiel externe,
dans lequel la zone d'antenne prédéterminée est plus petite que la zone de réflecteur prédéterminée.

4. Module de communication de données sans fil selon la revendication 3, dans lequel l'antenne NFC (105) est disposée sous le réflecteur optique (319) le long d'un axe longitudinal du module de communication de données sans fil (100) de telle sorte que le bord de réflecteur circonférentiel externe chevauche complètement le bord d'antenne circonférentiel externe le long de l'axe longitudinal.

5. Module de communication de données sans fil pour un dispositif d'injection de médicament selon l'une quelconque des revendications précédentes, dans lequel l'élément porteur flexible plié (107) comprend une carte de circuit imprimé flexible où la pluralité de régions de support de composant (203, 205, 207, 209, 211) sont formées de manière solidaire.

6. Module de communication de données sans fil pour un dispositif d'injection de médicament selon l'une quelconque des revendications 1 à 4, dans lequel l'élément porteur flexible plié (107) comprend une pluralité de sections de carte de circuit imprimé comprenant la pluralité de régions de support de composant (203, 205, 207, 209, 211), et
une pluralité d'éléments conducteurs flexibles et pliables électriquement interconnectant électriquement la pluralité de sections de circuit imprimé.

7. Module de communication de données sans fil selon l'une quelconque des revendications 3 à 6, dans lequel la zone d'antenne prédéterminée de l'antenne NFC est inférieure à 314 mm², de préférence inférieure à 180 mm² (d = 15 mm).

8. Module de communication de données sans fil selon l'une quelconque des revendications précédentes, dans lequel l'antenne NFC (105) comprend une pluralité d'enroulements de bobines formés comme des traces de fil de la deuxième région de support de composant (205) de l'élément porteur flexible plié (107) pour fournir une structure sensiblement plane de l'antenne NFC.

9. Module de communication de données sans fil selon la revendication 8, dans lequel la feuille perméable magnétiquement (106) et l'antenne NFC sensiblement plate (105) sont alignées l'une par rapport à l'autre dans une configuration de chevauchement.

10. Module de communication de données sans fil selon l'une quelconque des revendications 8 à 9, dans lequel la pluralité d'enroulements de bobines comprend :
- un premier ensemble d'enroulements de bobines formés sur une première surface de la deuxième région de support de composant (205) et un deuxième ensemble d'enroulements de bobines formés sur une deuxième surface de la deuxième région de support de composant (205), dans lequel la deuxième surface est disposée de manière opposée à la première surface sur la deuxième région de support de composant (205).

11. Module de communication de données sans fil selon l'une quelconque des revendications 1 à 10, dans lequel la feuille perméable magnétiquement (106) est fixée à une quatrième région de support de composant de l'élément porteur flexible plié (107), dans lequel la quatrième région de support de composant est située entre la deuxième région de support de composant (205) et la troisième région de support de composant (207).

12. Module de communication de données sans fil selon l'une quelconque des revendications 1 à 9, dans lequel la feuille perméable magnétiquement (106) est fixée à la deuxième région de support de composant (205) de l'élément porteur flexible plié (107), dans lequel la deuxième région de support de composant est située entre les première (203) et troisième (207) régions de support de composant protégeant l'antenne NFC du bruit EMI généré par l'ensemble de circuits électroniques.

13. Module de communication de données sans fil selon la revendication 12, dans lequel un espaceur (422) est fixé à une première surface de la feuille perméable magnétiquement (106) ; et
l'espaceur comprend un agent adhésif ou une pellicule (423) pour la fixation à la deuxième région de support de composant (205) de l'élément porteur flexible plié (107).

14. Module de communication de données sans fil selon l'une quelconque des revendications précédentes, comprenant en outre :
- une structure de boîtier cylindrique (601) comprenant un axe longitudinal central, une extrémité annulaire proximale et une extrémité distale, opposée, annulaire ;
dans lequel l'écran LCD ou OLED (319) est fixé au niveau de l'extrémité annulaire proximale avec l'écran transparent faisant face vers l'extérieur se projetant depuis la structure de boîtier cylindrique (601).

15. Dispositif d'injection de médicament en forme de stylo (600) comprenant :
une structure de boîtier en forme de stylo (601) ayant un élément de corps cylindrique creux qui s'étend entre une section de stylo distale et une section de stylo proximale, où la section de stylo proximale comprend une découpe de boîtier pour la réception d'une communication de données sans fil, et
un module de communication de données sans fil (100) selon la revendication 14 monté dans la découpe de boîtier.
